# EUROPEAN PATENT APPLICATION

(11) **EP 4 454 651 A1**
(43) Date of publication of application: **30.10.2024**
(21) Application number: 22911953.2
(22) Date of filing: 21.12.2022
(51) Int. Cl.: A61K 31/5365, A61K 36/268, A61P 35/00, A23L 33/10, C07D 498/04

(54) **USE OF ARISTOLOXAZINE C HAVING ANTICANCER ANTICITY**

(30) Priority: 21.12.2021 KR 20210184371
(71) Applicant: Korea Institute of Oriental Medicine, Daejeon 34054 (KR)
(72) Inventor: LEE, Jun, Jinju-si, Gyeongsangnam-do 52860 (KR); SEO, Young Hye, Naju-si, Jeollanam-do 58217 (KR); MOON, Byeong Cheol, Naju-si, Jeollanam-do 58217 (KR); LEE, A Yeong, Daejeon 34049 (KR); RYU, Seung Mok, Naju-si, Jeollanam-do 58217 (KR); KIM, Hyo Seon, Seoul 07614 (KR)
(74) Representative: Maiwald GmbH
(86) International application number: PCT/KR2022/021007
(87) International publication number: WO 2023/121320

(57) **Abstract**

The present invention relates to an anticancer use of aristoloxazine C, and more specifically to a pharmaceutical composition and a health functional food composition which use aristoloxazine C represented by Chemical Formula 1 or an isomer or pharmaceutically acceptable salt thereof; a method for preventing, ameliorating or treating cancer by using same; and a method for isolating aristoloxazine C.

## Description

### [Technical Field]

The present invention relates to the use of aristoloxazine C having anticancer activity, and more specifically to a pharmaceutical composition and a health functional food composition which use aristoloxazine C represented by Chemical Formula 1 below or an isomer or pharmaceutically acceptable salt thereof; a method for preventing, ameliorating or treating cancer by using same; and a method for isolating aristoloxazine C.

### [Background Art]

*Asarum heterotropoides* (Fr.Schmidt var. mandshuricum (Maxim.) Kitag.) belongs to the *Aristolochiaceae* family and is mainly distributed in Asia, including Korea, China and Japan. The roots and rhizomes of *A. heterotropoides* have been used in traditional medicine in some Asian countries to treat cough, headache, toothache, aphthous stomatitis, inflammation, local anesthesia and rheumatism [R. Zhou, "Resource science of Chinese medicinal materials", China Medical and Pharmaceutical Sciences Press, Beijing (1993); J. Lee et al., "Bioactive compounds from the roots of Asiasarum heterotropoides", Molecules 19 (1) (2014) 122-138]. In previous pharmacological studies, it was reported that the *Asarum heterotropoides* extract has anti-nociceptive, anti-inflammatory, acaricidal and larvicidal activities [Y. Xu et al., "Assessment on anti-nociception and anti-inflammation pharmacodynamics of Asarum heterotropoides var. mandshuricum and Asarum sieboldii", China J. Chin. Mat. Med. 37 (5) (2012) 625-631; J.-R. Kim et al., "Acaricidal activity of Asarum heterotropoides root-derived compounds and hydrodistillate constitutes toward Dermanyssus gallinae (Mesostigmata: Dermanyssidae)", Exp. Appl. Acarol. 68 (4) (2016) 485-495; H. Perumalsamy et al., "Larvicidal activity of Asarum heterotropoides root constituents against insecticide-susceptible and-resistant Culex pipiens pallens and Aedes aegypti and Ochlerotatus togoi", J. Agric. Food Chem. 58 (18) (2010) 10001-10006]. In addition, the essential oil of this plant has been reported to exhibit antibacterial and deodorizing activities [J. Moon, et al., "Physicochemical characterization and deodorant activity of essential oil recovered from Asiasarum heterotropoides using supercritical carbon dioxide and organic solvents", J. Ind. Eng. Chem. 69 (2019) 217-224; Y. Dan et al., "Activities of essential oils from Asarum heterotropoides var. mandshuricum against five phytopathogens", Crop Prot. 29 (3) (2010) 295-299]. Some phytochemical investigations on *Asarum heterotropoides* have revealed the presence of various types of lignans, monoterpenes, amides, flavanones, phenanthrenes, benzene derivatives and volatile phenylpropenes, which have useful biological effects such as anti-inflammatory, cytotoxic and anticancer activities [J. Lee et al., Bioactive compounds from the roots of Asiasarum heterotropoides, Molecules 19 (1) (2014) 122-138; Y. Jing et al., Chemical constituents from the roots and rhizomes of Asarum heterotropoides var. mandshuricum and the in vitro anti-inflammatory activity, Molecules 22 (1) (2017) 125; Y. Jing et al., Phenanthrene derivatives from roots and rhizomes of Asarum heterotropoides var. mandshuricum, Fitoterapia 117 (2017) 101-108; Y. Wang et al., Toxic and Repellent Effects of Volatile Phenylpropenes from Asarum heterotropoides on Lasioderma serricome and Liposcelis bostrychophila, Molecules 23 (9) (2018) 2131]. However, in spite of the traditional medical importance and biological effects, research on the anticancer activity of *Asarum heterotropoides* and related metabolites thereof is still insufficient.

Under this background, the inventors of the present invention completed the present invention by isolating the aristoloxazine C compound from the roots of *Asarum heterotropoides* and confirming its inhibitory effects on human cancer cell lines.

### [Disclosure]

### [Technical Problem]

Accordingly, an object of the present invention is to provide an anticancer composition using a compound isolated from *Asarum heterotropoides* roots.

With regard to the use, another object of the present invention is to provide a method for preventing, ameliorating or treating cancer using a compound isolated from *Asarum heterotropoides* roots.

With regard to the use, still another object of the present invention is to provide the use of a compound isolated from *Asarum heterotropoides* roots in the manufacture of a medicament or health functional food for preventing, ameliorating or treating cancer.

Still another object of the present invention is to provide a method for isolating a compound having anticancer activity from *Asarum heterotropoides* roots.

### [Technical Solution]

In order to solve the above-described problems, the present invention provides a pharmaceutical composition for preventing or treating cancer, including aristoloxazine C (Asaroidoxazine C) represented by Chemical Formula 1 below, an isomer or a pharmaceutically acceptable salt thereof as an active ingredient:

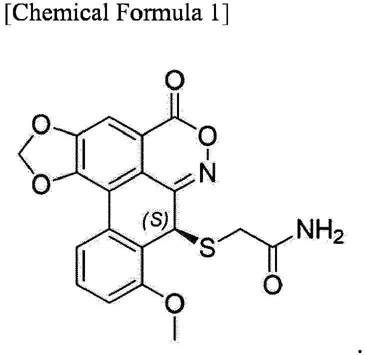

Additionally, the present invention provides a health functional food composition for preventing or ameliorating cancer, including the above-described compound, an isomer or a sitologically acceptable salt thereof as an active ingredient.

With regard to the use, the present invention provides a method for preventing, ameliorating or treating cancer, including the step of administering aristoloxazine C (Asaroidoxazine C) represented by Chemical Formula 1, an isomer or a pharmaceutically acceptable salt thereof to a subject in need thereof.

With regard to the use, the present invention provides the use of aristoloxazine C (Asaroidoxazine C) represented by Chemical Formula 1, an isomer or a pharmaceutically or sitologically acceptable salt thereof in the manufacture of a medicament or health functional food for preventing, ameliorating or treating cancer.

According to a preferred exemplary embodiment of the present invention, the isomer may include a racemate, an enantiomer, a diastereomer, a mixture of enantiomers or a mixture of diastereomers.

According to another preferred exemplary embodiment of the present invention, the aristoloxazine C may be isolated from *Asarum heterotropoides* roots.

According to still another preferred exemplary embodiment of the present invention, the aristoloxazine C, isomer or pharmaceutically or sitologically acceptable salt thereof may exhibit an effect of inhibiting cancer cells or inhibiting or delaying tumor development.

In addition, the present invention provides a method for isolating aristoloxazine C (Asaroidoxazine C) represented by Chemical Formula 1 from *Asarum heterotropoides* roots, including the steps of: 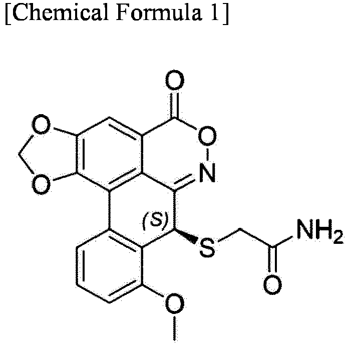
(a) extracting *Asarum heterotropoides* roots with an ethanol solvent to obtain an ethanol extract of the *Asarum heterotropoides* roots;
(b) obtaining an ethyl acetate fraction by systematically fractionating the ethanol extract of the *Asarum heterotropoides* roots in the order of hexane, ethyl acetate and water;
(c) performing flash chromatography on the ethyl acetate fraction by using a water-methanol mixture as an effluent solvent to obtain a fraction; and
(d) purifying the aristoloxazine C (Asaroidoxazine C) by subjecting the fraction obtained after performing column chromatography to semi-preparative chromatography by using a water-acetonitrile mixture as an effluent solvent.

According to a preferred exemplary embodiment of the present invention, the ethanol solvent in step (a) may be 50 to 90% ethanol.

According to another preferred exemplary embodiment of the present invention, the water-methanol mixture in step (c) may be applied at a concentration gradient of 6:4 to 1:9.

According to still another preferred exemplary embodiment of the present invention, the water-acetonitrile mixture in step (d) may be applied at a concentration gradient of 6.5:3.5 to 5.5:4.5.

### [Advantageous Effects]

Since aristoloxazine C provided in the present invention exhibits excellent anticancer activity even at a significantly lower concentration than 5-FU which is an existing anticancer drug, it can be effectively used for preventing, ameliorating or treating cancer, and it can prevent side effects from excessive use.

### [Description of Drawings]

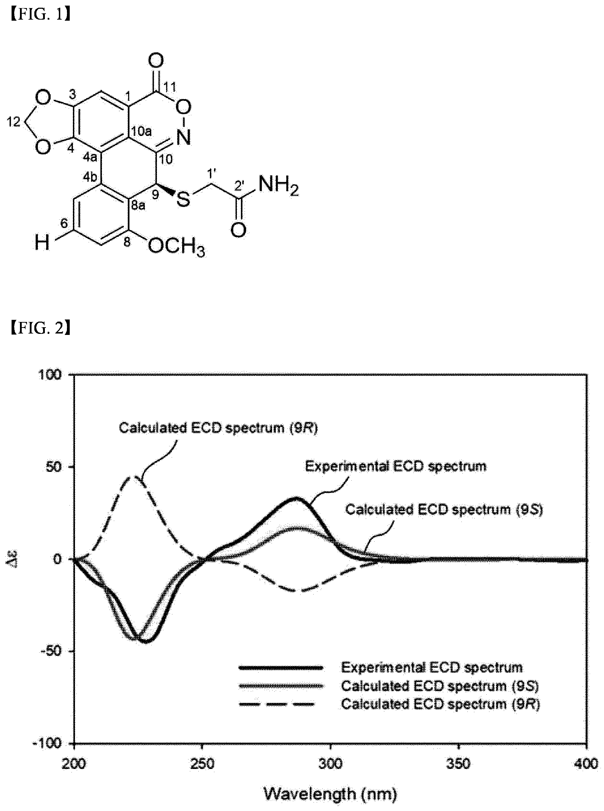

FIG. 1 shows the chemical structure of Compound 1 (Aristoloxazine C) isolated from *Asarum heterotropoides.*
FIG. 2 shows the computational and experimental ECD spectra of Compound 1 (Aristoloxazine C).

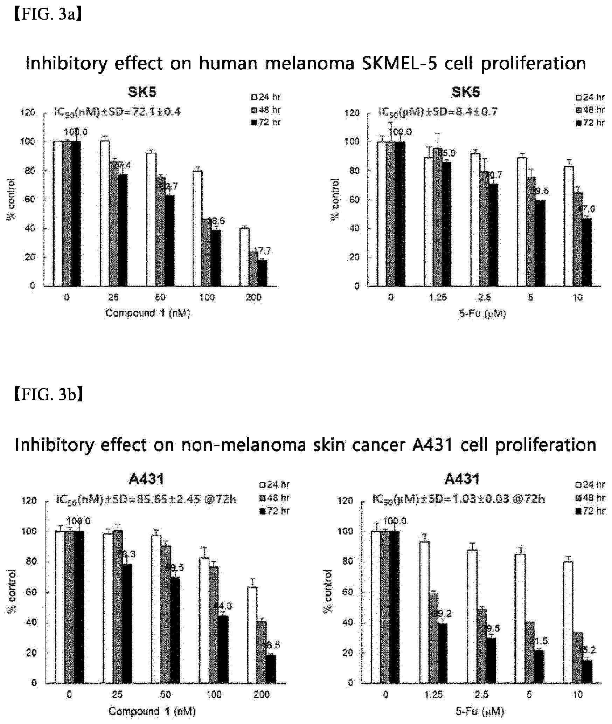

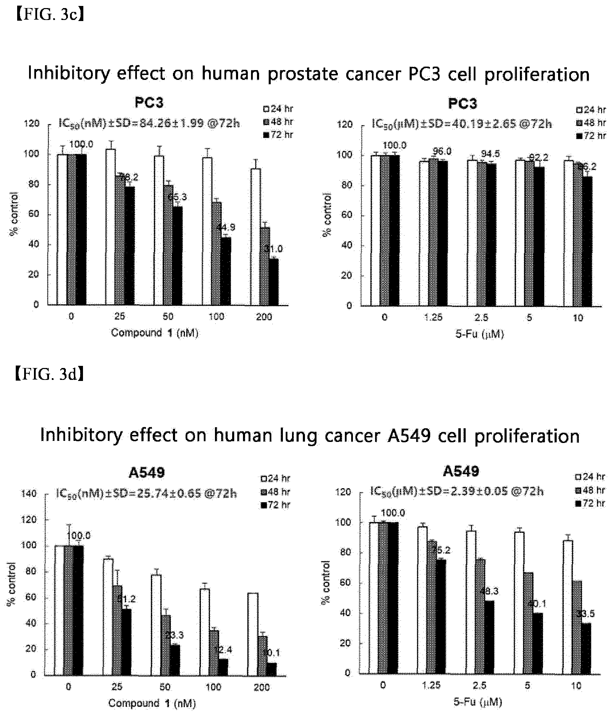

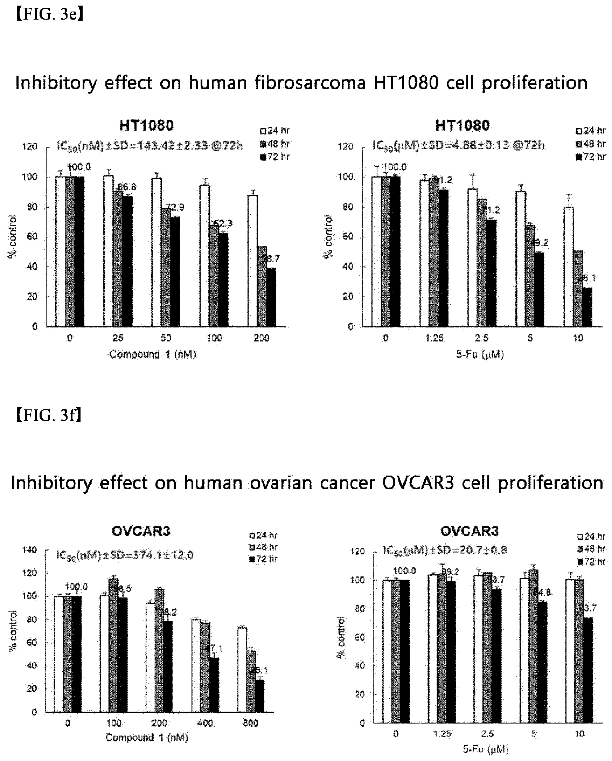

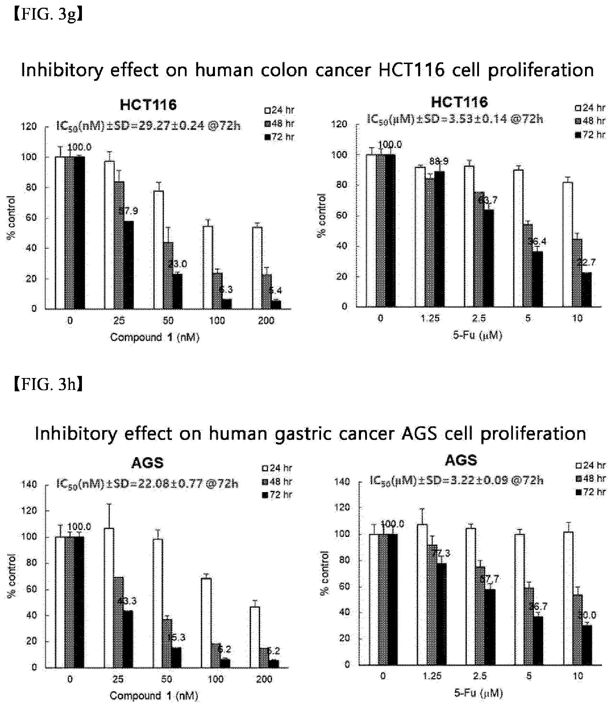

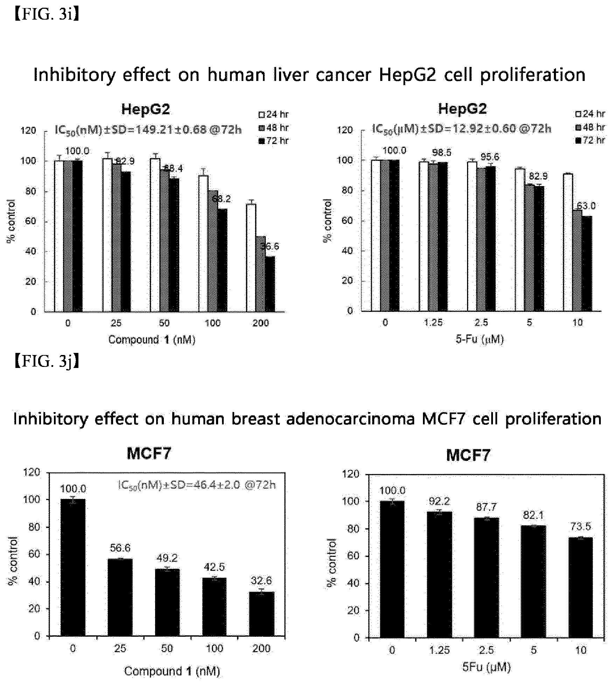

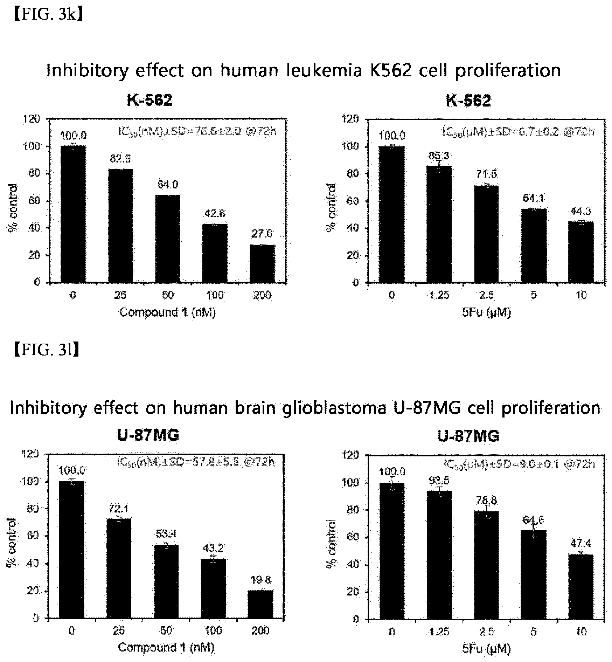

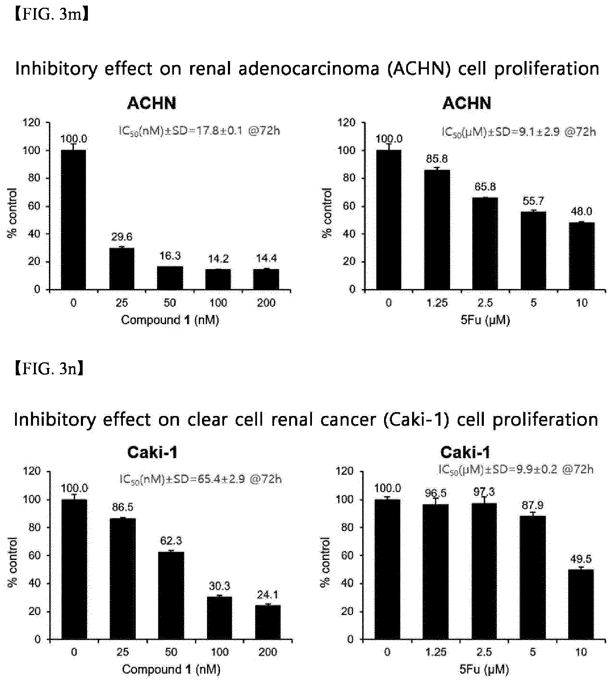

FIGS. 3a to 3n show the results of measuring the cell proliferation inhibitory effect of Compound 1 (Aristoloxazine C) on various cancer cell lines by MTT assay, wherein FIG. 3a shows the cell proliferation inhibitory effect on human melanoma cells, FIG. 3b shows the cell proliferation inhibitory effect on human non-melanoma skin cancer cells, FIG. 3C shows the cell proliferation inhibitory effect on human prostate cancer cells, FIG. 3D shows the cell proliferation inhibitory effect on human lung cancer cells, FIG. 3E shows the cell proliferation inhibitory effect on human fibrosarcoma cells, FIG. 3F shows the cell proliferation inhibitory effect on human ovarian cancer cells, FIG. 3G shows the cell proliferation inhibitory effect on human colon cancer cells, FIG. 3h shows the cell proliferation inhibitory effect on human stomach cancer cells, FIG. 3i shows the cell proliferation inhibitory effect on human liver cancer cells, FIG. 3j shows the cell proliferation inhibitory effect on human breast adenocarcinoma cells, FIG. 3k shows the cell proliferation inhibitory effect on human leukemia cells, FIG. 3I shows the cell proliferation inhibitory effect on human brain glioblastoma cells, FIG. 3m shows the cell proliferation inhibitory effect on human renal adenocarcinoma cells, and FIG. 3n shows the cell proliferation inhibitory effect on clear cell kidney cancer.
FIG. 4 is the results of evaluating the effect of Compound 1 (Aristoloxazine C) on inhibiting the growth of EGF-induced cell transformed colonies.

### [Best Mode]

Hereinafter, the present invention will be described in more detail.

Meanwhile, each description and exemplary embodiment disclosed herein may also be applied to each other description and exemplary embodiment. That is, all combinations of various elements disclosed herein fall within the scope of the present invention. In addition, it cannot be said that the scope of the present invention is limited by the specific description described below.

In addition, those skilled in the art will recognize or be able to ascertain using no more than routine experimentation, many equivalents to the specific exemplary embodiments of the invention described in the present application. In addition, such equivalents are intended to be included in the present invention.

As described above, the inventors of the present invention isolated the aristoloxazine C compound from the roots of *Asarum heterotropoides* and confirmed its excellent inhibitory effect on human cancer cell lines.

Accordingly, a first aspect of the present invention relates to a pharmaceutical composition for preventing or treating cancer, including aristoloxazine C (Asaroidoxazine C) represented by Chemical Formula 1 below, an isomer or a pharmaceutically acceptable salt thereof as an active ingredient; and a health functional food composition for preventing or ameliorating cancer, including aristoloxazine C (Asaroidoxazine C) represented by Chemical Formula 1 below, an isomer or a sitologically acceptable salt thereof as an active ingredient:

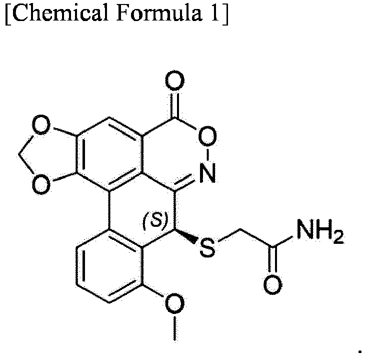

The *Asarum heterotropoides* as defined in the present invention may be *Asarum heterotropoides Fr. mandshuricum, Asarum sieboldi Miq* or *Asarum maculatum,* and preferably, it may be *Asarum heterotropoides Fr. mandshuricum.*

In an exemplary embodiment of the present invention, the isomer may include a racemate, an enantiomer, a diastereomer, a mixture of enantiomers or a mixture of diastereomers, but the present invention is not limited thereto.

In another exemplary embodiment of the present invention, the aristoloxazine C may be isolated from *Asarum heterotropoides,* and preferably, it may be isolated from roots. The *Asarum heterotropoides* may be purchased commercially, or those that are collected or cultivated in nature may be used.

Such cancers may be, for example, melanoma, non-melanoma skin cancer (e.g., squamous cell carcinoma), breast cancer, head and neck cancer, thyroid cancer, fibrosarcoma, soft tissue sarcoma, osteosarcoma, testicular cancer, prostate cancer, ovarian cancer, bladder cancer, skin cancer, brain cancer, glioma, hemangiosarcoma, mastocytoma, leukemia, lymphoma, liver cancer, lung cancer, pancreatic cancer, stomach cancer, kidney cancer, colon cancer, hematopoietic tumor, neuroblastoma, epidermal carcinoma or metastatic cancer thereof, but the present invention is limited thereto.

In a specific exemplary embodiment of the present invention, aristoloxazine C was isolated from the roots of *Asarum heterotropoides,* and the anticancer activity thereof was determined. As a result, as can be confirmed in Table 2 and FIGS. 3a to 3n, aristoloxazine C showed anti-proliferative activity against all cancer cells that were tested (melanoma, non-melanoma skin cancer, prostate cancer, lung cancer, fibrosarcoma, ovarian cancer, colon cancer, stomach cancer, liver cancer, breast cancer, leukemia, glioma and renal cancer). In particular, it was confirmed that it has significantly superior anticancer activity compared to the positive control 5-FU, which exhibits an inhibitory effect on cell proliferation at the micromolar (µM) level, by exhibiting an inhibitory effect on cell proliferation even at the nanomolar (nM) level.

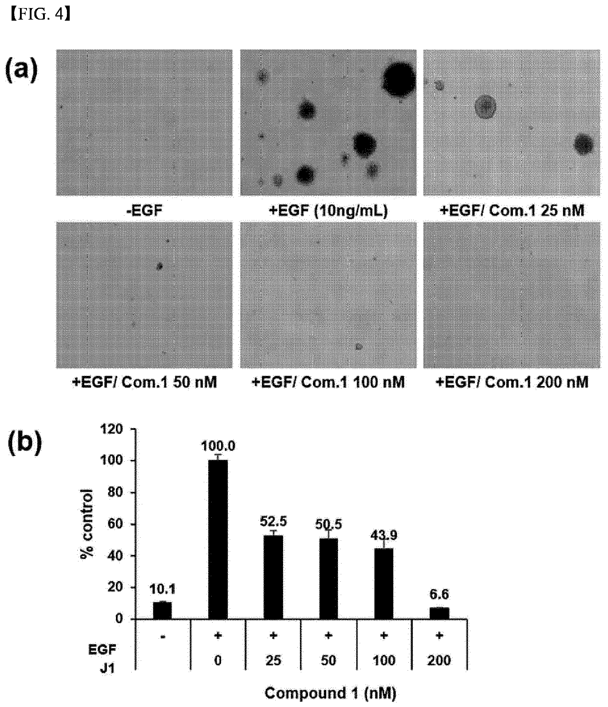

In another specific exemplary embodiment of the present invention, the anticancer activity of aristoloxazine C that was isolated from the roots of *Asarum heterotropoides* was evaluated in an epidermal growth factor (EGF)-induced carcinogenesis model. As a result, as can be confirmed in FIG. 4, aristoloxazine C exhibited an inhibition rate of 47.5 to 93.4% compared to the EGF-treated group in the EGF-induced carcinogenesis model, and the inhibition rate increased in a concentration-dependent manner of aristoloxazine C. Through this, it can be seen that aristoloxazine C exhibits cancer prevention effects by suppressing or delaying tumor development by inhibiting EGF-induced cell transformation.

Since the aristoloxazine C described above is isolated from *Asarum heterotropoides* roots, the *Asarum heterotropoides* root extract or fraction including the same may also be utilized for preventing, ameliorating or treating cancer.

As used herein, the term "extract" refers to an extract obtained by the extraction treatment of the *Asarum heterotropoides* roots, a diluted or concentrated solution of the extract, a dried product obtained by drying the extract, a crude purification product or a purified product of the extract, or a mixture thereof, and the extract itself and all formulations of extracts that can be formed by using the extract. The extract may be extracted from a natural, hybrid or mutant plant of *Asarum heterotropoides,* and it may also be extracted from a plant tissue culture.

The extract may be prepared by using hexane, chloroform, methylene chloride, ethyl acetate, acetone, C₁ to C₄ lower alcohol, water or a mixed solvent thereof as an extraction solvent, preferably, it may be prepared by using C₁ to C₄ lower alcohol, water or a mixed solvent thereof as an extraction solvent, but the present invention is not limited thereto.

In the composition of the present invention, the *Asarum heterotropoides* extract may be prepared by extracting the dried roots of *Asarum heterotropoides* at least once by using the extraction solvent, and it may be prepared as a dried extract obtained by concentrating the solvent extract under reduced pressure and then freeze-drying or spray-drying the same.

As used herein, the term "fraction" refers to a result obtained by performing fractionation in order to separate a specific component or a specific component group from a mixture including various components.

For example, the *Asarum heterotropoides* root fraction including aristoloxazine C may be prepared by performing the steps of (a) extracting *Asarum heterotropoides* roots with an ethanol solvent to obtain an *Asarum heterotropoides* ethanol extract; and (b) obtaining an ethyl acetate fraction by systematically fractionating the *Asarum heterotropoides* root ethanol extract in the order of hexane, ethyl acetate and water, but the present invention is not limited thereto. In step (a), the ethanol solvent may be 50 to 90% ethanol, preferably, 60 to 80% ethanol, and most preferably, 65 to 75% ethanol. When a solvent other than ethanol is used, the aristoloxazine C content is rapidly reduced, and thus, the intended anticancer activity may not be exhibited.

In the composition of the present invention, the term "prevention" means any action to suppress or delay the onset of a disease or condition. In the present invention, it means delaying the onset of cancer or suppressing the onset of cancer.

In the composition of the present invention, the term "amelioration" means any action that ameliorates or beneficially changes a disease or condition, and in the present invention, it means ameliorating the symptoms of cancer.

In the composition of the present invention, the term "treatment" means any action that delays, stops or reverses the progression of a disease or condition, and in the present invention, it means reducing, alleviating, eliminating or reversing the symptoms of cancer.

With regard to the use, the present invention further relates to a method for preventing, ameliorating or treating cancer, including the step of administering aristoloxazine C represented by Chemical Formula 1 above, an isomer or a pharmaceutically acceptable salt thereof to a subject in need thereof.

With regard to the use, the present invention further relates to the use of aristoloxazine C represented by Chemical Formula 1, an isomer or a sitologically acceptable salt thereof in the manufacture of a medicament or health functional food for preventing, ameliorating or treating cancer.

In terms of the methods and uses of the present invention, since the descriptions of aristoloxazine C, an isomer or a pharmaceutically or sitologically acceptable salt thereof and the effects thereof are the same as described above, the descriptions thereof will be omitted.

As used herein, the term "pharmaceutically acceptable salt" refers to a concentration that has an effective action that is relatively non-toxic and harmless to patients, and it refers to any organic or inorganic addition salt of the compound of Chemical Formula 1 in which the side effects resulting from this salt do not diminish the beneficial effects of the compound of Chemical Formula 1. For this salt, an inorganic acid and an organic acid may be used as free acids. As an inorganic acid, hydrochloric acid, bromic acid, nitric acid, sulfuric acid, perchloric acid, phosphoric acid and the like may be used. As an organic acid, citric acid, acetic acid, lactic acid, maleic acid, fumaric acid, gluconic acid, methanesulfonic acid, glycolic acid, succinic acid, tartaric acid, galacturonic acid, embonic acid, glutamic acid, aspartic acid, oxalic acid, (D) or (L) malic acid, maleic acid, methanesulfonic acid, ethanesulfonic acid, 4-toluenesulfonic acid, salicilic acid, citric acid, benzoic acid, malonic acid and the like may be used. In addition, these salts may include alkali metal salts (sodium salt, potassium salt, etc.) and alkali earth metal salts (calcium salt, magnesium salt, etc.). For example, the acid addition salt may include acetate, aspartate, benzoate, besylate, bicarbonate/carbonate, bisulfate/sulfate, borate, camcilate, citrate, edisylate, ethylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, methylate, methylsulfate, naphthalate, 2-naphthylate, nicotinate, nitrate, orotate, oxalate, palmitate, pamoate, phosphate/hydrogen phosphate/dihydrogen phosphate, saccharate, stearate, succinate, tartrate, tosylate, trifluoroacetate, aluminum, arginine, benzathine, calcium, choline, diethylamine, diolamine, glycine, lysine, magnesium, meglumine, olamine, potassium, sodium, tromethamine, zinc salt and the like. Among these, hydrochloride or trifluoroacetate may be used.

The pharmaceutical composition of the present invention may further include a pharmaceutically acceptable carrier.

The pharmaceutical composition of the present invention may be prepared into a pharmaceutical dosage form by using methods that are well-known in the art to provide a rapid, sustained or delayed release of the active ingredient after administration to a mammal. In the preparation of a dosage form, it is preferable to mix or dilute the active ingredient with a carrier or to encapsulate the same in a carrier in the form of a container.

Therefore, the pharmaceutical composition of the present invention may be used by formulating according to conventional methods into oral formulations such as powders, granules, tablets, capsules, suspensions, emulsions, syrups, aerosols and the like, external preparations, suppositories and sterile injection solutions, and it may further include appropriate carriers, excipients and diluents that are commonly used in the preparation of the composition.

For example, carriers that may be included in the pharmaceutical composition of the present invention include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil and the like, but the present invention is not limited thereto. When formulated, diluents or excipients such as fillers, extenders, binders, wetting agents, disintegrating agents, surfactants and the like that are commonly used are used for preparation.

Examples of solid preparations for oral administration include tablets, pills, powders, granules, capsules and the like, and in such solid preparations, at least one excipient, for example, starch, calcium carbonate, sucrose or lactose, gelatin and the like are mixed in the compound for preparation. In addition to simple excipients, lubricants such as magnesium stearate and talc are also used.

Examples of oral liquid preparations may include suspensions, solvents, emulsions, syrups and the like, and may include various excipients such as wetting agents, sweeteners, fragrances, preservatives and the like, in addition to commonly used simple diluents such as water and liquid paraffin.

Examples of formulations for parenteral administration include sterilized aqueous solutions, non-aqueous solvents, suspensions, emulsions, lyophilized formulations and suppositories. As the non-aqueous solvent and suspending agent, propylene glycol, polyethylene glycol, vegetable oils such as olive oil, injectable esters such as ethyl oleate and the like may be used. As the base of suppositories, witepsol, macrogol, tween 61, cacao butter, laurin butter, glycerogelatin and the like may be used.

As used herein, the term "administration" means introducing the pharmaceutical composition of the present invention to a patient by any suitable method.

The mode of administration of the pharmaceutical composition according to the present invention is not particularly limited and may be in accordance with a method conventionally used in the art. The mode of administration is not limited as long as it can reach the target tissue and may be intraperitoneal administration, intravenous administration, intramuscular administration, subcutaneous administration, intradermal administration, oral administration, topical administration and intranasal administration. The pharmaceutical composition according to the present invention may be prepared in various dosage forms depending on the desired mode of administration.

The pharmaceutical composition of the present invention may be administered in a pharmaceutically effective amount.

The "pharmaceutically effective amount" refers to an amount which is sufficient to treat a disease at a reasonable benefit/risk ratio applicable to medical treatment, and the effective dose level may be determined by considering factors including the type and severity of a subject, age, gender, the type of virus infected, the activity of the drug, sensitivity to the drug, the time of administration, the route of administration and the rate of excretion, the duration of treatment, drugs used concurrently and other factors well-known in the medical field.

Conventional daily dosages of the pharmaceutical composition according to the present invention may be appropriately selected by those skilled in the art and may be administered once or divided into several times.

The composition of the present invention may be administered daily or intermittently, and the number of administrations per day may be administered once or divided into two or three times. In addition, the composition of the present invention may be used alone or in combination with other drug treatments for immune enhancement. In consideration of all of the above factors, it is important to administer an amount that can obtain the maximum effect in a minimum amount without side effects, and it can be easily decided by those skilled in the art.

As used herein, the term "subject" refers to all animals, including humans, that have or are likely to develop metabolic diseases. In addition to humans, the animals may be mammals such as cows, horses, sheep, pigs, goats, camels, antelopes, dogs, cats and the like, which require treatment of similar symptoms, but the present invention is not limited thereto.

As used herein, the term "food" refers to meat, sausage, bread, chocolate, candy, snack, confectionery, pizza, ramen, other noodles, gum, dairy products including ice cream, various soups, beverages, tea, drinks, alcoholic beverages, vitamin complexes, health functional foods and health foods, and it includes all foods in a conventional sense.

The health functional food composition of the present invention may include forms such as pills, powders, granules, infusions, tablets, capsules, liquids and the like, and foods to which the composition of the present invention may be added include, for example, various foods, for example, drinks, gums, teas, vitamin complexes, dietary supplements and the like.

There is no particular limitation on other ingredients that may be included in the health functional food composition of the present invention other than containing aristoloxazine C and/or an *Asarum heterotropoides* extract including the same as an essential ingredient, and various herbal extracts, food supplement additives, natural carbohydrates or the like may be contained as additional components with conventional foods.

In addition, the food supplement additives include conventional food supplement additives in the art, for example, flavoring agents, flavors, coloring agents, fillers, stabilizers and the like.

Examples of the natural carbohydrates are monosaccharides, for example, glucose, fructose and the like; disaccharides, for example, maltose, sucrose and the like; and polysaccharides, for example, conventional sugar such as dextrin, cyclodextrin and the like, and sugar alcohol such as xylitol, sorbitol, erythritol and the like. Besides what is described above, for flavoring agents, natural flavoring agents (e.g., rebaudioside A, glycyrrhizin, etc.) and synthetic flavoring agents (saccharin, aspartame, etc.) may be used advantageously.

In addition to the above, the health functional food composition of the present invention may contain a variety of nutritional supplements, vitamins, minerals (electrolytes), flavoring agents such as synthetic flavoring agents, natural flavoring agents and the like, coloring agents and fillers (cheese, chocolate, etc.), pectic acid and a salt thereof, alginic acid and a salt thereof, organic acids, protective colloid thickeners, pH adjusters, stabilizers, preservatives, glycerin, alcohols, carbonation agents used in carbonated drinks and the like. Besides, it may contain pulp for the production of natural fruit juices, fruit juice drinks and vegetable drinks. These components may be used independently or in combination.

In the present invention, the health supplement food includes a health functional food, health food and the like.

The health functional food is the same term as food for special health use (FoSHU) and refers to foods that are processed to exhibit the bioregulatory function efficiently with high medical and medicinal effects in addition to nutrition supply. Herein, the term "functional" means controlling nutrients for the structure and function of the human body or obtaining useful effects for health use such as physiological actions and the like. The food of the present invention may be prepared by a method that is conventionally used in the art, and upon preparation, raw materials and ingredients conventionally added in the art may be added for preparation. In addition, the formulation of the food may also be prepared without limitation as long as the formulation is recognized as a food. The health functional food composition of the present invention may be prepared in various forms of formulations, and unlike general medicine, it has an advantage that there is no side effect and the like that may occur while taken for a long term because food is the raw material.

In the method of the present invention, the term "subject" includes any animal (e.g., human, horse, pig, rabbit, dog, sheep, goat, non-human primate, cow, cat, guinea pig or rodent), but the present invention is not limited thereto. These terms do not denote a specific age or gender. Accordingly, whether female or male, adult and neonatal subjects as well as fetuses are intended to be included. A patient refers to a subject with a disease or disorder. The term patient includes human and veterinary subjects.

In the method of the present invention, since the descriptions of aristoloxazine C, an isomer or a pharmaceutically or sitologically acceptable salt thereof and the effects thereof are the same as described above, the descriptions thereof will be omitted.

A second aspect of the present invention relates to a method for isolating aristoloxazine C represented by Chemical Formula 1 above from *Asarum heterotropoides* roots, including the steps of:
(a) extracting *Asarum heterotropoides* roots with an ethanol solvent to obtain an ethanol extract of the *Asarum heterotropoides* roots;
(b) obtaining an ethyl acetate fraction by systematically fractionating the ethanol extract of the *Asarum heterotropoides* roots in the order of hexane, ethyl acetate and water;
(c) performing flash chromatography on the ethyl acetate fraction by using a water-methanol mixture as an effluent solvent to obtain a fraction; and
(d) purifying the aristoloxazine C by subjecting the fraction obtained after performing column chromatography to semi-preparative chromatography by using a water-acetonitrile mixture as an effluent solvent.

In the method of the present invention, the *Asarum heterotropoides* roots of step (a) may be dried through a conventional drying method such as natural drying or hot-air drying method.

In the method of the present invention, in the step (a), the type of extraction solvent used to extract the *Asarum heterotropoides* roots is ethanol. Extraction using ethanol (i.e., alcohol extraction) is the most eco-friendly and economical extraction method in food processing. Preferably, 50% to 90% ethanol, more preferably, 60% to 80% ethanol, and most preferably, 65% to 75% ethanol may be used as the extraction solvent.

The method for preparing the extract is not particularly limited, and it may be extracted according to a method commonly used in the art. Non-limiting examples of the extraction method include immersion extraction, hot water extraction, ultrasonic extraction, filtration, reflux extraction and the like, which may be performed alone or in combination of two or more methods.

In the method of the present invention, step (c) may be to perform flash chromatography by applying a water-methanol mixture at a concentration gradient of 6:4 to 1:9, and preferably, a standard reversed-phase hydrophobic resin, for example, a column packed with C18 resin may be used to perform flash chromatography.

In the method of the present invention, the water-acetonitrile mixture in step (d) may be applied at a concentration gradient of 6.5:3.5 to 5.5:4.5 to perform semi-preparative chromatography, and preferably, a standard reversed-phase hydrophobic resin., for example, a column packed with ODS-A resin may be used to perform semi-preparative chromatography.

Hereinafter, the present invention will be described in more detail by the following examples. However, the following examples are only for exemplifying the present invention, and the scope of the present invention is not limited thereto.

### [Modes of the Invention]

### [Example 1]

### Preparation of Asarum heterotropoides extract and isolation of aristoloxazine C compound

### 1-1. General procedure

Optical rotation and UV spectra were determined by using an MCP-5500 digital polarimeter (Anton Paar, Graz, Austria) and an Optizen POP spectrophotometer (Mecasys, Daejeon, Korea), respectively. Electronic circular dichroism (ECD) spectra were recorded on a JASCO J-1100 spectropolarimeter (Jasco, Tokyo, Japan). NMR spectra were acquired on an AVANCE III 600 MHz Cryo-NMR (Bruker, Billerica, MA, USA). HRESIMS data were acquired by using a Bruker compact or Waters Q-TOF mass spectrometer (Waters, Milford, MA, USA). Preparative HPLC was performed by using a Waters prep-LC system consisting of a 2998 PDA detector and a 2545Q gradient module equipped with a YMC-Pack ODS-A semi-preparative column (21.2 × 250 mm, 5 µm, 120 Å, YMC, Kyoto, Japan). Column chromatography was performed through a Selekt flash chromatography system (Biotage, Uppsala, Sweden) provided with Biotage SNAP Ultra 100 g and SNAP Ultra C18 120 g pre-packed cartridges and 10 g Biotage SNAP dry rod cartridges manually packed with Sephadex LH-20 gel (MilliporeSigma, St. Louis, MO, USA).

### 1-2. Extraction and isolation

Dried *Asarum heterotropoides* roots were purchased from KMD Medicinal Herbs Co. in 2019 (Ulsan, South Korea) and deposited at the Herbal Medicine Resources Research Center of the Korea Institute of Oriental Medicine (Voucher Number 2-19-0399).

The dried *Asarum heterotropoides* roots (1 kg) were ground, macerated at 40°C and extracted with 70% ethanol (10 L), and then, these were filtered and evaporated in vacuum to obtain a total extract (86.3 g, 8.6%). This extract (80.0 g) was suspended in distilled water (1.5 L) and partitioned with a series of organic solvents to obtain n-hexane-soluble (9.3 g), EtOAc-soluble (2.7 g) and aqueous extracts (66.9 g). The active EtOAc extract was separated into two SNAP Ultra C18 120 g cartridges (water/MeOH, 6:4 to 1:9) by using a flash chromatography system (FCS) to obtain 23 fractions (F01-F23). F14 (18.7 mg) was purified by YMC-Pack ODS-A semi-preparative columns (water/CH 3 CN, 6.5:3.5 to 5.5:4.5, 50 min) to generate Compound 1 (4.9 mg).

**Aristoloxazine C (Compound 1):** Yellowish crystals; ECD (c 0.5 mM, CH₃CN) Δ*ε* +3.97 (289), -5.44 (223); ¹H (DMSO-*d*₆, 600 MHz) and ¹³C (DMSO-*d*₆, 150 MHz) NMR data are shown in Table 1.

**[Table 1]**

| position | 1^{a} | |
|---|---|---|
| | *δ*_{C}, type | *δ*_{H}, multi. (*J* in Hz) |
| 1 | 118.2, C | |
| 2 | 105.3, CH | 7.62, s |
| 3 | 153.5, C | |
| 4 | 150.0, C | |
| 4a | 113.0, C | |
| 4b | 127.7, C | |
| 5 | 119.0, CH | 7.92 d (8.0) |
| 6 | 130.2, CH | 7.53, t (8.1) |
| 7 | 112.7, CH | 7.22, d (8.3) |
| 8 | 156.3, C | |
| 8a | 119.5, C | |
| 9 | 38.2, CH | 5.43, s |
| 10 | 151.8, C | |
| 10a | 117.6, C | |
| 11 | 162.9, C | |
| 12 | 104.2, CH₂ | 6.45, s, 6.55, s |
| 1' | 34.4, CH₂ | 3.20, d (15.0), 3.32, m^{b} |
| 2' | 169.5, C | |
| NH₂-2' | | 7.14, br s, 7.35, br s |
| OCH₃-6 | | |
| OCH₃-7 | | |
| OCH₃-8 | 56.2, CH₃ | 3.95, s |

| | | |
|---|---|---|
| ^{a} Measured at DMSO-*d*₆. ^{b} Signals partially overlap. | | |

### 1-3. Computational methods

Conformer distribution, optimization and ECD calculations were performed as described in [Ryu, Seung Mok et al. "Chemical Constituents of the Egg Cases of Tenodera angustipennis (Mantidis ootheca) with Intracellular Reactive Oxygen Species Scavenging Activity." Biomolecules vol. 11,4 556. 10 Apr. 2021].

Compound 1 was obtained as yellow crystals, and molecular ions were observed at m/z 421.0467 [M + Na]⁺ in HRESIMS, respectively. The 1D NMR spectrum of Compound 1 was similar to the spectrum of aristoloxazine C. Aristoloxazine C is an aristolochic acid derivative including an oxazine ring, and it has recently been reported to be isolated from the genus *Asarum.* The absolute configuration at position C-9 of Compound 1 was determined through comparison between experimental and calculated ECD spectra. As confirmed in FIG. 4, the experimental ECD spectrum was in good agreement with the calculated ECD spectrum of the 9S model. Based on this spectroscopic evidence, the structure of Compound 1 was determined to be aristoloxazine C.

### [Example 2]

### Confirmation of anticancer activity through MTT assay

In order to evaluate the anticancer activity of aristoloxazine isolated in Example 1, the cytotoxic activity against 14 human cancer cell lines including SKMEL5 (melanoma), A431 (non-melanoma skin), PC3 (prostate), A549 (lung), HT1080 (fibrosarcoma), OVCAR3 (ovary), HCT116 (colon), AGS (stomach), HepG2 (liver), MCF7 (breast cancer), K562 (leukemia), U-87 (glioma), ACHN (renal adenocarcinoma) and Caki (clear cell kidney cancer) was evaluated.

Cells were seeded (1 to 2.5 × 10³ cells per well) in 96-well plates and then allowed to settle overnight. Next, the cells were treated with different doses of the compound for 24 hours, 48 hours or 72 hours in a 37°C CO₂ incubator, and 20 µL of MTT ((4,5-dimethylthiazol-2-yl)-2,5-diphenyltetrazolium bromide reagent (Sigma-Aldrich, Inc., St. Louis, MO, USA) was added to each well. After 1 to 2 hours, the medium was discarded, and 100 µL of DMSO (dimethyl sulfoxide, VWR Life Science, Ohio, USA) was added, and then, absorbance was measured at 570 nm by using a Multiskan Skyhigh microplate spectrophotometer (Thermo Scientific, Singapore). 5-FU was used as a positive control. The extract in Table 2 was a 70% ethanol *Asarum heterotropoides* root extract obtained in the process of preparing the *Asarum heterotropoides* root fraction of Example 1-2.

**[Table 2]**

| **Cell Line** | IC₅₀ Value (72h) | | |
|---|---|---|---|
| | **Aristoloxazine C** (nM) | **Extract** (ng/mL) | **5-FU** (µM) |
| NHDF (normal skin) | n.d. | n.d. | n.d. |
| SKMEL5 (melanoma) | 72.1±0.4 | n.d. | 8.4±0.7 |
| A431 (non-melanoma skin cancer) | 85.7±2.5 | n.d. | 1.0±0.03 |
| PC3 (prostate cancer) | 84.3±2.0 | n.d. | n.d. |
| A549 (lung cancer) | 25.7±0.7 | n.d. | 2.40±0.1 |
| HT1080 (fibrosarcoma) | 143.4±2.3 | n.d. | 4.9±0.1 |
| OVCAR3 (ovarian cancer) | 374.1±12.0 | n.d. | n.d. |
| HCT116 (colon cancer) | 29.3±0.2 | n.d. | 3.5±0.1 |
| AGS (stomach cancer) | 22.1±0.8 | n.d. | 3.2±0.1 |
| HepG2 (liver cancer) | 149.2±0.7 | n.d. | n.d. |
| MCF7 (breast cancer) | 46.4±2.0 | - | n.d. |
| K562 (leukemia) | 78.6±2.0 | - | 6.7±0.2 |
| U-87 (glioma) | 57.8±5.5 | - | 9.0±0.2 |
| ACHN (renal adenocarcinoma) | 17.8±0.1 | - | 9.1±2.9 |
| Caki (clear cell kidney cancer) | 65.4±2.9 | - | 9.9±0.2 |

| | | | |
|---|---|---|---|
| n.d.: not detected | | | |

As a result, as shown in Table 2 and FIGS. 3a to 3n, aristoloxazine C exhibited strong inhibitory activity with a much stronger nanomolar IC₅₀ value than 5-FU in all cancer cell lines.

Specifically, aristoloxazine C exhibited cytotoxicity against all cells that were tested with IC₅₀ values between 17.8 and 374.1 nM. In particular, the IC₅₀ values of AGS, A549, HCT116, MCF7 and ACHN cells were 22.1 ± 0.8, 25.7 ± 0.7, 29.3 ± 0.2, 46.4 ± 2.0 and 17.8 ± 0.1 nM, respectively, which showed the strongest activity.

### [Example 3]

### Confirmation of cancer prevention effect in EGF-induced carcinogenesis model

The cancer-inducing inhibitory effect of aristoloxazine C was determined in a carcinogenesis model in which malignant transformation was induced by treating mouse skin epithelial cell JB6 (ATCC, USA) with EGF, which is a growth factor that increases cancer cell development.

Specifically, an agar mix (0.5%) including BME, FBS, glutamine, gentamycin, PBS and 1.25% agar was mixed with EGF (10 ng/mL) and aristoloxazine C at each concentration, and 3 mL per well was dispensed into a 6-well plate and solidified (bottom agar). Mouse skin epithelial cells JB6 (ATCC, USA) were detached by treatment with trypsin, washed with 1X PBS and resuspended in 10% BME to confirm the number of cells. 8,000 cells per well were prepared and mixed with agar mix (0.3%), EGF and aristoloxazine C by concentration (0, 25, 50, 100 and 200 nM, respectively), and 1 mL per well was dispensed and solidified (upper agar). After culturing in a CO₂ incubator for about 2 weeks, colonies were photographed under a microscope, and the number of colonies was counted by using Image-Pro Plus software (v.6.0) program (Media Cybernetics, Silver Spring, MD, USA).

As a result, as can be confirmed in FIG. 4, the test group which was treated with aristoloxazine C exhibited an inhibition rate of 47.5 to 93.4% compared to the EGF-treated group in the EGF-induced carcinogenesis model, and it was confirmed that the inhibition rate of aristoloxazine C increased in a concentration-dependent manner. Through this, it was confirmed that aristoloxazine C has an effect of inhibiting or delaying tumor development.

From the above description, those skilled in the art to which the present invention pertains will be able to understand that the present invention may be embodied in other specific forms without changing the technical spirit or essential features thereof. In this regard, the exemplary embodiments described above should be understood as illustrative in all respects and not restrictive. The scope of the present invention should be construed as including the meaning and scope of the patent claims described below rather than the detailed description above, and all changes or modified forms derived from the equivalent concept thereof are included in the scope of the present invention.

## Claims

1. A pharmaceutical composition for preventing or treating cancer, comprising aristoloxazine C (Asaroidoxazine C) represented by Chemical Formula 1, an isomer or a pharmaceutically acceptable salt thereof as an active ingredient:

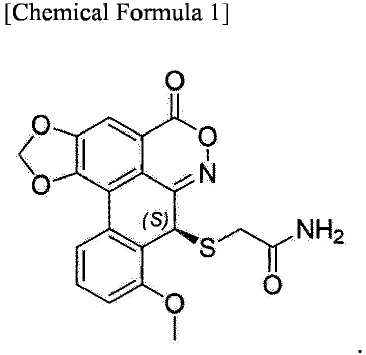

2. The pharmaceutical composition of claim 1, wherein the isomer includes a racemate, an enantiomer, a diastereomer, a mixture of enantiomers or a mixture of diastereomers.

3. The pharmaceutical composition of claim 1, wherein the aristoloxazine C is isolated from *Asarum heterotropoides* roots.

4. The pharmaceutical composition of claim 1, wherein the aristoloxazine C, isomer or pharmaceutically acceptable salt thereof exhibits an effect of inhibiting cancer cells or inhibiting or delaying tumor development.

5. A health functional food composition for preventing or ameliorating cancer, comprising aristoloxazine C (Asaroidoxazine C) represented by Chemical Formula 1, an isomer or a sitologically acceptable salt thereof as an active ingredient:

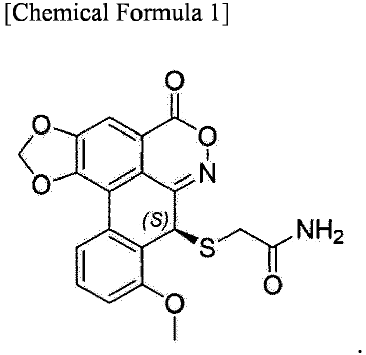

6. The health functional food composition of claim 5, wherein the isomer includes a racemate, an enantiomer, a diastereomer, a mixture of enantiomers or a mixture of diastereomers.

7. The health functional food composition of claim 5, wherein the aristoloxazine C is isolated from *Asarum heterotropoides* roots.

8. The health functional food composition of claim 5, wherein the aristoloxazine C, isomer or pharmaceutically acceptable salt thereof exhibits an effect of inhibiting cancer cells or inhibiting or delaying tumor development.

9. A method for isolating aristoloxazine C (Asaroidoxazine C) represented by Chemical Formula 1 from *Asarum heterotropoides* roots, comprising the steps of: 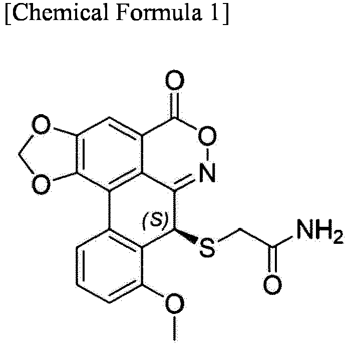
(a) extracting *Asarum heterotropoides* roots with an ethanol solvent to obtain an ethanol extract of the *Asarum heterotropoides* roots;
(b) obtaining an ethyl acetate fraction by systematically fractionating the ethanol extract of the *Asarum heterotropoides* roots in the order of hexane, ethyl acetate and water;
(c) performing flash chromatography on the ethyl acetate fraction by using a water-methanol mixture as an effluent solvent to obtain a fraction; and
(d) purifying the aristoloxazine C (Asaroidoxazine C) by subjecting the fraction obtained after performing column chromatography to semi-preparative chromatography by using a water-acetonitrile mixture as an effluent solvent.

10. The method of claim 9, wherein the ethanol solvent in step (a) is 50 to 90% ethanol.

11. The method of claim 9, wherein the water-methanol mixture in step (c) is applied at a concentration gradient of 6:4 to 1:9.

12. The method of claim 9, wherein the water-acetonitrile mixture in step (d) is applied at a concentration gradient of 6.5:3.5 to 5.5:4.5.

13. A method for preventing, ameliorating or treating cancer, comprising the step of administering aristoloxazine C (Asaroidoxazine C) represented by Chemical Formula 1, an isomer or a pharmaceutically acceptable salt thereof to a subject in need thereof:

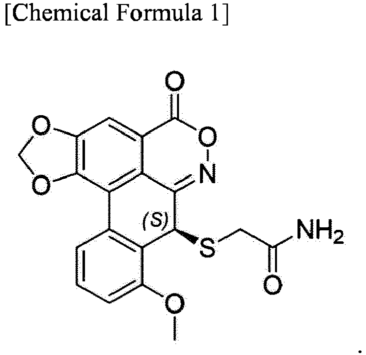

14. The method of claim 13, wherein the isomer includes a racemate, an enantiomer, a diastereomer, a mixture of enantiomers or a mixture of diastereomers.

15. The method of claim 13, wherein the aristoloxazine C is isolated from *Asarum heterotropoides* roots.

16. The method of claim 13, wherein the aristoloxazine C, isomer or pharmaceutically acceptable salt thereof exhibits an effect of inhibiting cancer cells or inhibiting or delaying tumor development.

17. Use of aristoloxazine C (Asaroidoxazine C) represented by Chemical Formula 1, an isomer or a pharmaceutically or sitologically acceptable salt thereof in the manufacture of a medicament or health functional food for preventing, ameliorating or treating cancer:

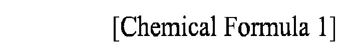

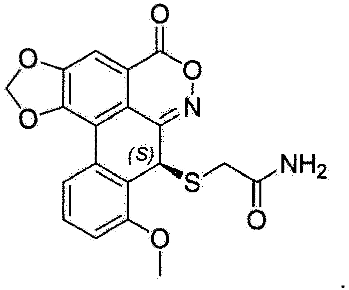

18. The use of claim 17, wherein the isomer includes a racemate, an enantiomer, a diastereomer, a mixture of enantiomers or a mixture of diastereomers.

19. The use of claim 17, wherein the aristoloxazine C is isolated from *Asarum heterotropoides* roots.

20. The use of claim 17, wherein the aristoloxazine C, isomer or pharmaceutically or sitologically acceptable salt thereof exhibits an effect of inhibiting cancer cells or inhibiting or delaying tumor development.
